# EUROPEAN PATENT APPLICATION

(11) **EP 4 379 046 A1**
(43) Date of publication of application: **05.06.2024**
(21) Application number: 22217331.2
(22) Date of filing: 30.12.2022
(51) Int. Cl.: C12N 5/0793

(54) **A 3D CELLULAR MODEL OF EARLY DIABETIC RETINOPATHY**

(30) Priority: 30.11.2022 PT 2022118368
(71) Applicant: UNIVERSIDADE NOVA DE LISBOA, 1099-085 Lisboa (PT)
(72) Inventor: NOGUEIRA TENREIRO, Sandra, Isabel, 1150-082 LISBOA (PT); DE LEMOS MACHADO, Maria, Luísa, 1150-082 LISBOA (PT); PIRES CARDOSO DE SEABRA, Miguel, Pedro, LISBOA 1150-082 (PT)
(74) Representative: Pereira da Cruz, Joao

(57) **Abstract**

The present invention discloses an *in vitro* human 3D cellular model comprising induced pluripotent stem cells (iPSCs) expressing BRN3B, CRX, RCVRN, VEGF and IL-1β for treating or preventing a disease or disorder of the eye in a patient, wherein the 3D cellular model mimics an early stage of said disease or disorder of the eye. A process for obtaining a pharmaceutical preparation comprising the step of providing a 3D cellular model is also disclosed. The present disclosure also relates to the 3D model of the present invention for drug screening or gene therapy and pre-clinical and clinical research, for preventing and treating neurodegeneration and inflammation, preferably in early DR and for personalized medicine treatment.

## Description

### Field of the Invention

The present invention is enclosed in the area of *in vitro* cellular culture development for novel therapeutic testing or pre-clinical research for novel anti-eye diabetic drugs for diabetic retinopathy (DR). It is also within the scope of the present invention a process for development of gene therapy approaches.

Such model uses differentiated retinal organoids from human induced Pluripotent Stem Cells (hiPSCs) to generate a disease model of early DR, that mimics several features of the human retina in a diabetic eye.

### Background of the Invention

There is an increasing interest in finding alternatives to animal testing, because, in addition to being costly, time-consuming and ethically questionable, data from animals frequently fails to predict the results obtained in human clinical trials. The lack of human-relevant preclinical models and the resulting high failure rates of therapeutics in the clinic has led to an unsustainable rise in healthcare costs as well as fewer effective drugs reaching patients. There is additionally increasing pressure from the society and governments to find alternatives to animal testing, as evidenced by the US Food and Drug Administration (FDA) Modernization Act of 2021 and the Humane Research and Testing Act (HR 1744), which are currently moving through the US Congress.

Increase in incidence and prevalence of chronic diseases as diabetes, adoption of organoids as therapeutics tools, application of organoids in drug discovery or gene therapy & personalized medicine, and use of organoids in research & development is anticipated to propel the global organoids market. The global organoids market was valued over US$ 1.7 Bn in 2019 and is expected to cross the market value of US$ 12.8 Bn by the end of 2030. DR is a major complication of diabetes and a leading cause of visual impairment in the working-age population. The global DR market size was valued at USD 6.6 billion in 2021 and is expected to grow at a compound annual growth rate (CAGR) of 6.4% from 2022 to 2030. The rising prevalence of diabetes, growing geriatric population, and increasing prevalence of blindness due to diabetes are among the major factors anticipated to boost market growth over the forecast period.

Diabetic retinopathy (DR) is a major complication of diabetes and a leading cause of visual impairment in the working-age population affecting around30% of the patients after 5 years of diabetes diagnosis.

DR is a retinal degenerative disorder wherein hyperglycemia leads to morphological and cell functional impairment of specific retinal cell types. The therapeutic strategies available for DR only target the later stages of the disease involving vascular defects such as macular edema or neovascularization. However, retinal neurodegeneration and inflammation is known to precede vascular alterations.

The DR complex models developed so far involve animal experimentation and present several limitations on pre-clinical studies, increasing therefore the rate of failure during clinical stages, which ultimately translate into higher costs for a novel drug to treat and cure diabetic patients with visual loss.

Nowadays, it is clinically accepted that addressing DR in the early phase is crucial to find a cure to treat patients with DR, a chronic disease.

Importantly, the therapeutic strategies available for DR only target the later stages of the disease, slowing disease progression but they do not heal. Therefore, it is urgent to identify new therapeutic approaches acting on early stages and, consequently, halting disease progression. Thus, DR market leaders face increased competition from new drug launches and new management treatment approval from regulatory bodies, making it difficult for them to sustain themselves in the ever-changing market. However, heavy investments in R&D conducted by market leaders are said to boost the market growth.

There are several technologies known to date addressing this problem, namely animal models on DR, 2D cellular models and human explant models.

The present solution provides an advantageous technical effect in view of animal models on DR because it uses no animal model (in agreement with the 3Rs for animal research), it mimics a human model - increasing the chances of success on clinical trials phases and is less expensive.

The present solution provides a second advantageous technical effect in view of 2D cellular models because it reproduces the complexity and stratification of the human retina, reproducing both de neuronal and the glial phenotypes of the early stage of the disease.

The present solution provides yet another advantageous technical effect in view of human explant models because it is easier to obtain, involving no ethical issues and is easier to maintain in culture.

Solutions exist in the art where, such as the case of patent application GB2584664A which discloses an improved retinal organoids and methods of making the same; WO2020223226A1 disclosing compositions and methods for the treatment of retinal degeneration; US2022042975A1 disclosing methods of generating organoids for high throughput screening of drugs; EP1306426 disclosing *in vitro* micro-organs, and uses related thereto; EP2338980 disclosing regeneration and repair of neural tissue using postpartum umbilical cord -derived cells; EP2566566 disclosing adult stem cells/progenitor cells and stem cell proteins for treatment of eye injuries and diseases and document WO02064748 - Multipotent Adult Stem Cells, Sources Thereof, Methods Of Obtaining And Maintaining Same, Methods Of Differentiation Thereof, Methods Of Use Thereof And Cells Derived Thereof.

However, due to the complexity of the retinal tissue, the known existing solutions present the following issues:
- 2D cellular models of DR are too simple lacking the retina complexity, leading to high rate of false positive during drug discovery or gene therapy.
- organotypic retinal cultures (retinal explants) conserve the histotypic context of the retina but present several technical limitations such as the duration and the viability of the in vitro culture.
- human models are often subject to time-consuming and challenging ethical consents.
- rodent models lack a cone-rich macula and therefore, a human eye disease model cannot be accurately established in mouse.
- non-human primate models present high structural similarity to the human eyes, but they have a high cost regarding the housing requirements, imposed limitations on research and ethical on pre-clinical studies, such as risk of translational failure to the humans.

The present solution intended to innovatively overcome such issues, by reproducing a higher level of complexity in the human cellular model and additionally considering the early stages of the chronic disease.

### General Description

The present disclosure relates to a 3D disease model which reproduces the main molecular and cellular features of the early phase of DR. The present disease-mimicking tool is extremely powerful for pharmaceutical drug development process targeting DR, from drug discovery or gene therapy to preclinical research.

In an embodiment, the present technology is used for selecting the most promising drug candidates for clinical trials.

In an embodiment, the present technology uses differentiated retinal organoids from human induced Pluripotent Stem Cells (hiPSCs), which resemble the cellular organization of the human retina, which is known in the art. Those organoids are then exposed to a diabetic microenvironment to generate a disease model of early DR.

With the model of the present invention, it is possible to reproduce established molecular and cellular features of early DR, such as the loss of ganglion and amacrine cells, the induction of glial reactivity and inflammation, together with the increase of VEGF and IL-1β expression and MCP-1 secretion. Moreover, increased levels of reactive oxygen species (ROS) accompanied by activation of key enzymes involved in antioxidative stress response is also obtained with the present model.

Thus, the 3D-organoid disease model for DR provides a valuable and exceptional tool to explore the early neurodegeneration mechanism which occurs before vascularization problems. The reproducibility in the present 3D model of retinal organoids opens new perspectives to its application in developing targeted therapeutic strategies against neurodegeneration and inflammation in early DR, consequently delaying the progression of the disease, which addresses an unmet clinical need in DR, providing a technical solution to an existing problem in the art.

In an embodiment, the 3D organoid-based technology of the present invention is a better tool for companies targeting DR drug discovery or gene therapy and pre-clinical research because it presents the following advantages:
the 3D in vitro model of the present disclosure creates the cellular organization of the human retina in a diabetic eye, resulting in a remarkable accurate model for drug development that allows most promising candidates being translated into clinical trials;
human iPSCs from healthy individuals (commercially available) are used to induce the diabetic organoid, therefore existing no need for cells from diabetic patients with DR;
the existence of an organoid-based technology for DR avoids and reduces the use of animals on pre-clinical studies. Moreover, mouse eye is very different from human eye, the present model providing a reliable model which mimics the human eye;
the present technology is scalable: a large quantity of organoids that can be obtained from human iPSCs and created on a dish is unlimited when compared with commonly used animal models, limited to two eyes per animal;
organoids disease models are an effective time- and cost-reducing tool in drug screening and preclinical research for biotech and pharmaceutical industry.

In an embodiment, the present invention represents an *in vitro* disease model of DR, reproducing an early stage of this chronic disease, which represents a landmark in the present field research.

In order to develop the *in vitro* disease model for DR, a previous established protocol for human retinal organoids was used as a starting point to differentiate hiPSCs (IMR90-4) and subsequently, generate a model of early DR by optimizing its exposition to a diabetic microenvironment.

Several cycles of optimization and validation were performed using conditions that induce the diabetic environment in cells and tissues. Hallmarks of early DR were used to fine-tune and to reach the best conditions that mimic the diabetic eye in early stages of the disease. Namely, the stage of retinal organoids development, the duration of the treatment and the concentration of glucose to be used were carefully optimized.

Thus, in the 3D model of the present invention, loss of ganglion and amacrine cells are observed by exposing cell to high glucose levels. Glial reactivity and inflammation were also reproduced, with increased expression of the VEGF and IL-1β encoding genes, and enhanced MCP-1 secretion. Moreover, there are increased levels of reactive oxygen species (ROS) accompanied by activation of key enzymes such as superoxide dismutase (SOD) and catalase, involved in antioxidative stress response. These changes are all well-established molecular and cellular features of early DR, confirming that an *in vitro* human 3D-organoid model for early DR was created for the first time.

The 3D-organoid model for DR is industrially applicable. It is an object of the present invention a product formulated as cryopreserved solution, for example supplied in frozen cryovials; organoid lines ready for seeding into plate assays with growth conditions established with consistency and reproducibility.

It is also within the scope of the present invention a process for drug screening and pre-clinical research.

The capability of 3D-organoid cultures to mimic organ functionality make them an extremely valuable tool in personalized medicine treatment, for evaluation of clinical response to treatment - Patient in the lab, as well as drug discovery (clinical trials on a dish), allowing informed decisions in early stages of the drug development pipeline, saving therefore time and costs.

The present model is also useful for viral and non-viral gene therapy and pre-clinical testing, by modulating the expression of factors of therapeutic interest for DR delay or treatment.

Moreover, integration of the 3D-organoids technology in the drug development process significantly improves the chance of success in clinical trials.

It is also an object of the present invention developing an organoid-on-a-chip system with a microfluidic device and/or incorporating microglia, a vascular system, or retinal pigments epithelium on retinal organoids in one of the objects of the present invention.

For purposes of interpretation within the scope of the present disclosure, the term "organoid" shall be interpreted as an artificially grown mass of cells or tissue that resembles an organ.

In a preferred embodiment, the present invention discloses an *in vitro* human 3D cellular model comprising induced pluripotent stem cells (iPSCs) expressing BRN3B, CRX, RCVRN, VEGF and IL-1β for treating or preventing a disease or disorder of the eye in a patient, wherein the 3D cellular model mimics an early stage of said disease or disorder of the eye.

In a further embodiment, the present invention discloses an 3D cellular model according to the present invention for treating or preventing Diabetic Retinopathy.

In a further embodiment, the present invention discloses an 3D cellular model according to the present invention comprising from 5-10 AP2_{α}⁺ cells/section (%) of amacrine cells when exposed to levels of glucose from 50-70 mM and MCP-1 secretion from 1.2-2 ng/ml MCP-1/µg protein and fluorescence intensity levels of intracellular reactive oxygen species from 11-14 arbitrary units.

In a further embodiment, the present invention discloses a process for obtaining a pharmaceutical preparation comprising the step of providing a 3D cellular model according to the present invention.

In a further embodiment, the present invention discloses a process for obtaining the 3D cellular model of the present invention comprising the step of obtaining differentiated retinal organoids from human iPSCs to generate an early-stage model of a given eye disease or disorder, preferably diabetic retinopathy.

In a further embodiment, the present invention discloses an 3D cellular model according to the present invention for drug screening or gene therapy and pre-clinical and clinical research.

In a further embodiment, the present invention discloses an 3D cellular model according to the present invention for preventing and treating neurodegeneration and inflammation, preferably in early DR.

In a further embodiment, the present invention discloses an 3D cellular model according to the present invention for personalized medicine treatment.

In a further embodiment, the present invention discloses a system comprising the 3D cellular model according to the present invention, preferably comprising a microfluidic device, microglia, a vascular system and/or retinal pigments epithelium on retinal organoids.

In a further embodiment, the present invention discloses a system comprising the 3D cellular model according to the present invention being an organoid-on-a-chip.

None of the objects of the present invention involve the use of human embryos as base material.

### Brief Description of Drawings

The following Figures are shown as a representation of particular embodiments and shall not be regarded as limiting the scope of the disclosure:

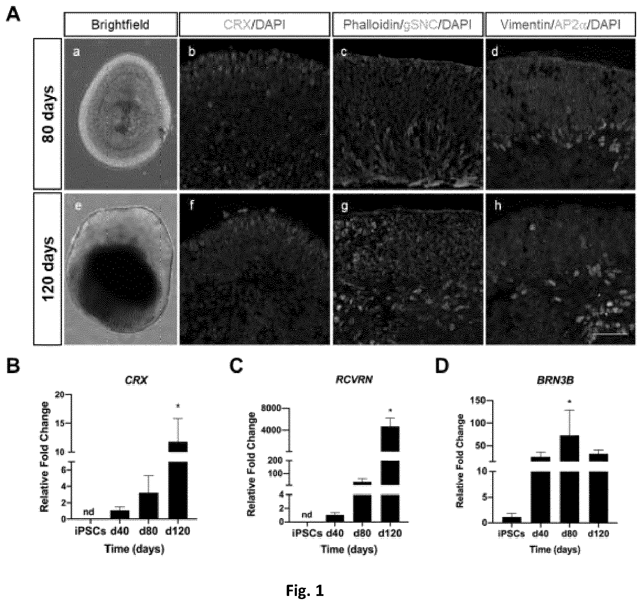

**Figure 1** depicts a possible embodiment of the retinal organoids derived from human iPSCs at 80 days and 120 days of differentiation. A shows brightfield images (a and e) and immunofluorescence staining of CRX, Phalloidin, gSNC, Vimentin, AP2α counterstained with DAPI (b to h) at a scale bar of 50 µm. In B to D, the gene expression analysis is of, respectively, BRN3B, CRX and RCVRN at iPSCs stage, day 40, day 80 and day 120 is shown. Data is presented as Mean ± SD from 3 independent differentiations and differences were considered statistically significant at *p<0.05.

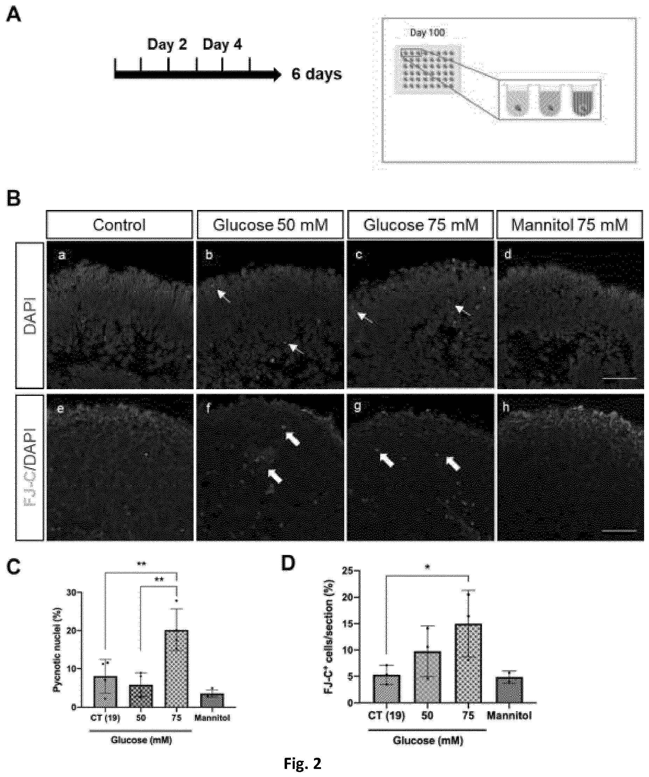

**Figure 2** shows a configuration of the present invention where high glucose treatment induces neurodegeneration in day 100 (d100) retinal organoids. A depicts a schematic representation of the experimental protocol where retinal organoids at day 100 were exposed to 50 and 75 mM of D-Glucose for 6 days, whereas control organoids were maintained in normal media with 19mM of glucose and the osmotic control was represented by 75mM of Mannitol. In B, cross-sectional images of retinal organoids with DAPI nuclei staining (in thin arrows) showing pyknotic nuclei (a-d) and cross-sectional images of retinal organoids with FluoroJade-C (FJ-C) (e-h) staining (in large arrows). Arrows indicate pyknotic nuclei (thin) and degenerating neurons (thick). The quantification of pyknotic nuclei after high glucose treatment is shown in C and the quantification of FJ-C positive cells (FJ-C⁺) after high glucose treatment in D. Statistical results are the mean values ± SD of 2-3 organoids from at least 3 independent differentiations (*p value<0.05 and **p value<0.01).

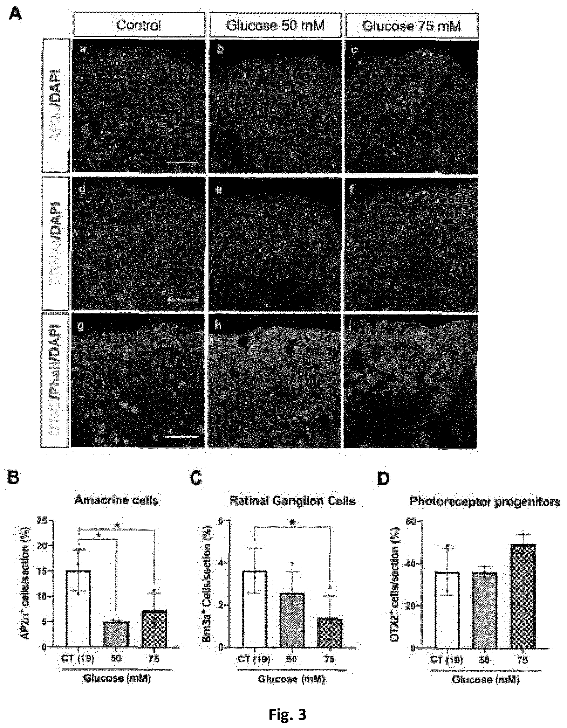

**Figure 3** illustrates an embodiment of the present invention where high glucose conditions induce alterations on different retinal cell types. Immunofluorescence analysis of organoids subjected to high glucose (a-c) staining of AP2α (amacrine cells), (d-f) staining of BRN3a (retinal ganglion cells) and (g-i) staining of OTX2 (photoreceptor progenitors) and Phalloidin (outer limiting membrane) is shown in A. Organoids sections were counterstained with DAPI. Figure 3 also depicts the quantification of AP2α-positive cells counted per field per field of view (B), the quantification of Brn3a-positive cells counted per field per field of view (C) and the quantification of OTX2-positive cells counted per field of view (D). Data represents mean ± SD of at least 3 independent differentiations (*p value<0.05).

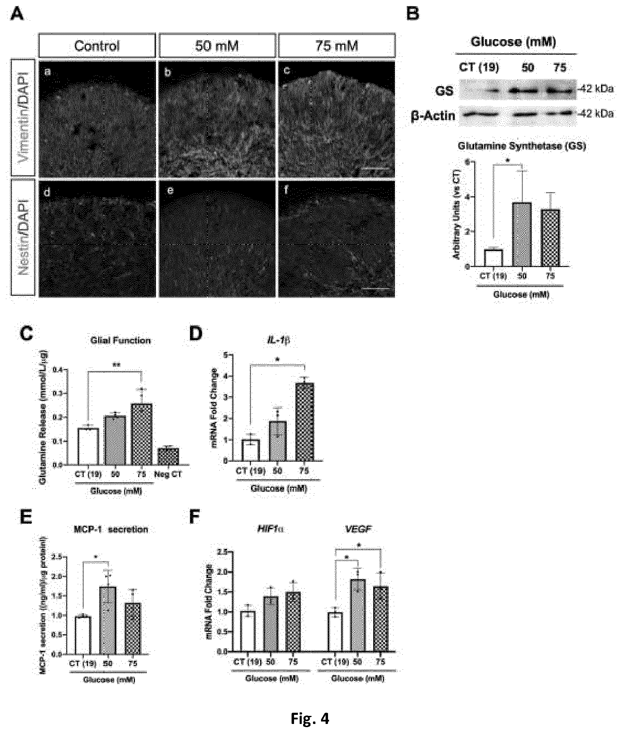

**Figure 4** shows an embodiment of the present invention where hyperglycemia conditions induce an inflammatory response in retinal organoids. The immunofluorescence staining of Vimentin (a-c) and Nestin (d-f) counterstained with DAPI (dark grey) is shown in A Figure 4 also shows a western blot analysis of Glutamine Synthase (GS) (upper panel) and correspondent densitometry analysis of GS normalized by β-Actin (lower panel) (B), the glutamine release in the culture medium after high glucose treatment, reflecting the conversion of glutamate to glutamine by the glial cells (C), the expression of *HIF1α* and *VEGF* mRNA (D), the expression of the pro-inflammatory cytokine *IL-1β* mRNA (E) and the MCP-1 secretion levels in the culture medium measured by ELISA (F). Results represent the mean±SD of at least 3 independent differentiations (^{∗}*p* value<0.05 and ^{∗∗}*p* value<0.01).

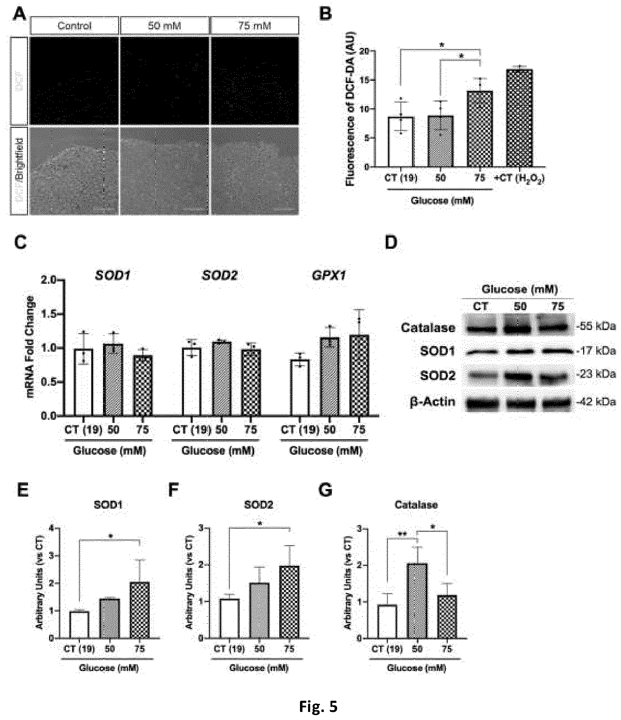

**Figure 5** embodies a scenario of the present invention where high glucose conditions affect the antioxidant response in organoids. Confocal images of DCF-DA (2,7- dichlorofluorescein diacetate) probe for detection of reactive oxygen species in organoids after high glucose treatment (A). Figure 5 also shows the quantification of the fluorescence intensity of DCF-DA (n=4) (B), the expression of SOD1, SOD2 and GPX1 mRNA levels (C), the western blot analysis of catalase and superoxide dismutase 1 and 2 (SOD1 and SOD2) (D) and the densitometry analysis of Catalase, SOD1 and SOD2 normalized by β-Actin (E-G). Data represent the mean±SD of at least 3 independent differentiations (^{∗}*p* value<0.05 and ^{∗∗}*p* value<0.01).

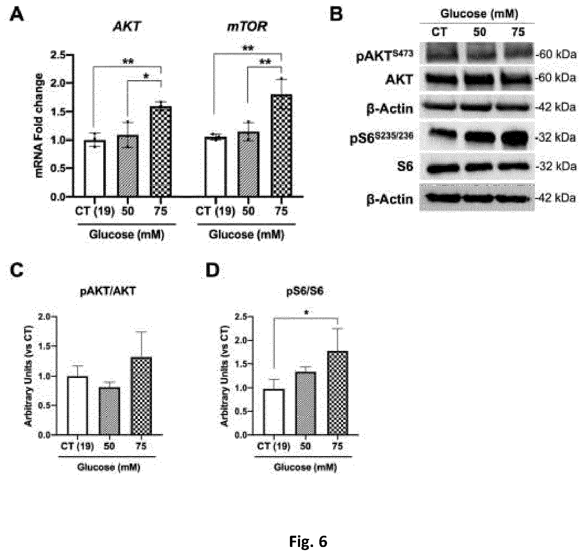

**Figure 6** shows the effects of hyperglycemia conditions on mTOR signalling pathway. The expression levels of AKT and mTOR mRNA levels after high glucose treatment (A), western blot analysis of phospho-AKTS473, AKT, phospho-S6S235/236 and S6 protein levels normalized by β-Actin (B), densitometry of pAKT/AKT levels after glucose treatment (C), densitometry of pS6/S6 protein levels after high glucose conditions are shown. Data represent mean±SD of at least 3 independent differentiations (^{∗}*p* value<0.05 and ^{∗∗}*p* value<0.01).

### Detailed Description of the Invention

It is an object of the present invention to provide a human 3D-organoid model for simulating the early stage of DR.

It is simultaneously an object of the present invention to provide a process for drug screening and pre-clinical research.

In a preferred embodiment, the human 3D-organoid model for simulating the early stage of DR comprises:
i) Commercially available hiPSC cultures;
ii) Retinal organoid differentiation of iPSCs;
iii) High glucose conditions.

The validation of the suitability of the present model for early DR was successfully carried out by cell death and inflammation detection, immunofluorescence analysis, gene expression and protein levels of different markers of interest.

### Example

In an example, the hiPSC line (IMR90-4, WiCell) is cultured with mTeSR^{™} Plus Medium (STEMCELL Technologies) on growth factor reduced matrigel-coated 6-well plates (Corning). The hiPSCs are routinely passaged using Versene (Gibco, Thermo Fisher Scientific) at a ratio of 1:4-1:6 in mTeSR^{™} Plus medium supplemented with 10 µM of ROCK inhibitor Y-27632 (Focus Biomolecules) and maintained at 37 ºC in a humidified atmosphere containing 5% CO₂.

The method for differentiating the hiPSCs cells towards retinal organoids was carried out by:
i) lifting the hiPSCs at 80% of confluence using Versene (Gibco, Thermo Fisher Scientific) and splitting at 3 000 cells/well in a Nunclon Sphera 96-well U-bottom plate (Thermo Fisher Scientific) using mTeSRTM Plus medium supplemented with 10 µM of ROCK inhibitor (Focus Biomolecules).
ii) growing the cells as aggregates/embryoid bodies for 6 days, doing an adaptation to Neural Induction Medium (NIM) containing DMEM/F12, 1% N2 supplement, 1X Non-Essential Amino Acids, 1X GlutaMax (all from Gibco, Thermo Fisher Scientific) and 2 mg/ml Heparin (Sigma-Aldrich).
iii) on day 6, adding 1.5 nM of Bone Morphogenic Protein-4 (BMP4, Peprotech) to fresh NIM, and on day 7 EBs (Embryoid bodys) transferring to growth factor reduced matrigel-coated 6-well plates (Corning).
iv) replacing the medium by half fresh NIM on days 9, 12 and 15.
v) on day 16, replacing the medium by retinal differentiation medium (RDM) containing DMEM:F12 (3:1), 2% B27 minus vitamin A, 1X Non Essential Amino Acids, 1X GlutaMax, 1X Antibiotic/Antimycotic (all form Gibco, Thermo Fisher Scientific) and changing every 2-3 days.
vi) on day 30, manually dissecting the artificial optic vesicles using a surgical scalpel (SM65A, Swann-Morton Ltd) under the microscope EVOS XL core (Thermo Fisher Scientific). After dissection, organoids were maintained in suspension flasks (Sarstedt) in 3D-retinal differentiation medium (3D-RDM) containing DMEM:F12 (3:1), 2% B27 minus vitamin A, 1X Non-Essential Amino Acids, 1X GlutaMax, 1X Antibiotic/Antimycotic, 5% FBS, 1:1000 chemically defined lipid supplement (all from Gibco, Thermo Fisher Scientific), 100 µM taurine (Sigma-Aldrich) and 1 µM of all-trans retinoic acid (RA, Sigma-Aldrich) until day 100.
vii) conditioning the organoids in 3D-RDM and processed for high glucose treatments.

At a later stage, the method of creating high glucose conditions, optionally and preferably, includes:
i) exposing the organoids at day 100 to different glucose concentrations. By supplementing the 3D-RDM with D-Glucose (Sigma-Aldrich) to a final concentration of preferentially 50 mM and 75 mM.
ii) preserving the control organoids in the 3D-RDM medium at standard concentration of 19 mM of glucose.
iii) replacing the preservation medium the organoids described in i) every other day for a total of 6 days.
iv) using D-Mannitol (Sigma-Aldrich) to a final concentration of 75 mM for osmotic control for osmotic control.

In a preferred embodiment, the human 3D-organoid model for simulating the early stage of DR is validated by:
i) Measuring the intracellular reactive oxygen species (ROS) levels
ii) Assaying the glutamine release
iii) Histology and Immunofluorescence
iv) Fluoro-Jade C staining
v) Western blot analysis
vi) RNA extraction and Quantitative real-time PCR
vii) Enzyme-Linked Immunosorbent Assay (ELISA)

Preferably, the ROS are measured using the cell permeable fluorogenic probe 2',7'-Dichlorodihydroflurescein diacetate (DCF-DA, Sigma-Aldrich), widely used to determine the degree of overall oxidative stress. In detail, the preferred embodiment of the present invention, is a method which, after the glucose treatment:
i) Incubates the organoids in 3D-RDM medium with 20 µM of DFC-DA for 1h at 37ºC;
ii) Does positive control testing using 0.5% H₂O₂ for 30 minutes;
iii) rinses the organoids once in PBS and plated in chamber slides for live imaging using confocal microscope Zeiss LSM 980 (Zeiss). The mean fluorescence intensity per image is preferably determined using Fiji (Image J) software.

In a preferred embodiment, the assaying of the glutamine release or glial function are quantified as the intracellular conversion of L-Glutamate to L-Glutamine. L-Glutamic acid (Sigma) was added at 3 mM in GlutaMax-free 3D-RDM medium at the third day of glucose treatment. Medium culture samples are optionally collected at day 6, centrifugated at 200 g, 4 ºC for 10 min and supernatants are collected for glutamine analysis. Glutamine quantifications are measured using Cedex Bio analyzer 7100 (Roche), and concentrations are normalized by the protein content of each sample (mmol/L/µg).

An example for validating the histology and immunofluorescence methodology is described as follows:
i) fixating the retinal organoids in 4% Paraformaldehyde phosphate-buffer solution (PFA, Sigma) for 20 minutes at room temperature, washing twice with phosphate-buffered saline (PBS) and incubating in 10% for 1h, 20% for 1h, and 30% sucrose solution overnight at 4 ºC.
ii) placing said retinal organoids into cryogenic moulds and immersing them in optimum cutting temperature (OCT Cryomatrix, Fisher Scientific).
iii) sectioning preferably 12 µM thick slices on a Leica CM3050 S cryostat (Leica), while permeabilizing cut sections with 0.25% Triton-X-100 (Sigma-Aldrich) in PBS for 15 minutes and blocking in 10% Donkey Serum (DS) in PBS for 1 hour.
iv) Incubating primary antibodies in blocking solution at 4 ºC overnight. Primary antibodies to use in the described methodology are not limited to:
   mouse anti-AP2α (1:100, sc-12726, Santa Cruz Biotechnology), goat anti-BRN3a (1:100, sc-31989, Santa Cruz Biotechnology), goat anti-OTX2 (1:100, AF1979, R&D Systems), mouse anti-CRX (1:200, H00001406-M02, Abnova),
   rabbit anti-SNCG (1:200, ab55424, Abcam), rabbit anti-Vimentin (1:200, 5741, Cell Signalling), mouse anti-Nestin (1:200, MAB353, Chemicon), Alexa Fluor 568 Phalloidin (1:400, A12380, Invitrogen).
v) washing the sections three times with PBS followed by incubating the secondary antibodies for 1 hour at room temperature. Examples of the secondary antibodies are not limited to (all from Invitrogen and used at 1:1000 dilution): Donkey anti-goat 488 (A11055), Donkey anti-mouse 488 (A21202), Donkey anti-rabbit (A21206), Donkey anti-mouse 568 (A10037) and Donkey anti-rabbit 488 (A10042).
vi) incubating the slices with 4', 6-diamidino-2-phenylindole (DAPI, 1 µg/ml, Sigma-Aldrich), washing twice with PBS and mounted with Vectashield mounting medium (Vector Laboratories).
vii) Confocal images are preferably acquired using a Zeiss LSM 980 (Zeiss) and images were processed preferably using Image J Software.

Preferably, the current method uses Fluoro-Jade C (FJC) as an anionic fluorochrome commonly used for identifying degenerating neurons in the brain and in the retina regardless the neurotoxic insult or the mechanism of cell death. In a preferred embodiment, the identification is carried out as follows:
i) performing FJC labelling on retinal organoids cryosections using the Fluoro-Jade^{®} C staining kit (Biosensis).
ii) incubating FJC and 4,6-diamidino-2-phenylindole (DAPI), air dry the solution for 10 minutes, clear in in xylene and mount with the EntellanTM (Sigma-Aldrich) mounting media
iii) analysing samples preferably in the confocal microscope Zeiss LSM980 (Zeiss) and expressing the number of FJC+ cells relative to the total number of DAPI stained nuclei (at least five sections per condition were analysed of three independent experiments).

Preferably, the method for protein detection is carried out as following:
i) resuspending pools of 2 to 4 organoids in cold lysis buffer (Cell Signalling Technology) supplemented with protease and phosphatase inhibitor cocktails (Roche) and sonicated twice for 3 seconds at 10% intensity in the Branson digital Sonifier SFX 150 (Emerson).
ii) centrifugating the Lysates at 7500 g for 10 minutes at 4 ºC, and collecting supernatant for protein quantification using the Pierce BCA protein assay kit (Thermo Scientific) following the manufacturer's instructions.
iii) Resolving a total of 20 µg of protein from each lysate on 10% or 12% sodium dodecyl sulphate - polyacrylamide gels (SDS-PAGE) and subsequently transferring to nitrocellulose membranes (Bio-Rad Laboratories).
iv) blocking the membranes using 5% non-fat dry milk or 5% bovine serum albumin (BSA) (Sigma-Aldrich) in Tris-buffered saline (TBS) (50 mM Tris, 150 mM NaCl, pH = 7.6) containing 0.1% Tween-20 (Sigma-Aldrich) (TBS-T).
v) incubating primary antibodies in the blocking solution overnight at 4ºC. Examples of primary antibodies suitable for the presented method are: rabbit anti-Glutamine Synthetase (1:1000, NB110-41404, Novus Biologicals), mouse anti-Catalase (1:500, sc-271803, Santa Cruz Biotechnology), mouse anti-SOD1 (1:1000, sc-31989, Santa Cruz Biotechnology), rabbit anti-SOD2 (1:1000, ab13533, Abcam) , rabbit anti-phospho-Akt Ser473 (1:1000, 9271, Cell Signaling Technology), rabbit anti-Akt (1:1000, 9272, Cell Signaling Technology), rabbit anti-phospho-S6 Ribosomal protein Ser235/236 (1:1000, 4858, Cell Signaling Technology), rabbit anti-S6 Ribosomal Protein (1:1000, 2217, Cell Signaling Technology), mouse anti-β-Actin-peroxidase (1:25000).
vi) washing the solution with TBS-T and adding the appropriate HRP-conjugated secondary antibody (1:5000 in blocking buffer) for 2 h at room temperature. Secondary antibodies suitable for use are not limited to: donkey anti-rabbit HRP (NA934, Cytiva) and sheep anti-mouse HRP (NA931, Cytiva).
vii) Detecting the antibody binding using chemiluminescence ECL Prime Western Blotting Substrate (Cytiva) and preferably acquiring images on ChemiDoc Touch (Bio-Rad Laboratories). The acquired images can be processed and quantified using Image Lab software (Bio-Rad laboratories) and the protein of interest is normalized using β-Actin as a loading control.

The preferred method for extracting the RNA and quantitative real-time PCR includes the following steps:
i) Isolating the mRNA from 2 to 4 organoids using the RNeasy mini kit (Qiagen) according to manufacturer's protocol.
ii) Reverse transcribing 1 µg of mRNA into cDNA using Superscript II reverse transcriptase kit (Invitrogen).
iii) using the cDNA samples for quantitative PCR in the LightCycler 96 system (Roche) using the FastStar Essential DNA Green Master (Roche) following the manufacturer's instructions.
iv) designing primers pairs using for example Primer-BLAST (NCBI) and synthesizing by Thermo Fisher Scientific. The following primer sequences are suitable for the described purpose (forward/reverse): AKT, (5'-TGATCACCATCACACCACCT-3' / 5'-CTGGCCGAGTAGGAGAACTG-3'); GPX, (5'-TGGGCATCAGGAGAACGCCA-3'/ 5'-GCGTAGGGGCACACCGTCAG-3'); HIF1α, (5'-CAGTCGACACAGCCTGGATA-3'/5'-GCGGCCTAAAAGTTCTTCTG-3'); IL1B, (5'-GTTTCTCTGCAGAAAGAGGC-3'/5'-AATGCCAGAGATGCATTGG-3'); mTOR, (5'-CTGGTTTCACCAAACCGTCT-3'/5'-GCACGACGTCTTCCAGTACC-3'); SOD1, (5'-TGGCCGATGTGTCTATTGAA-3'/5'-ACCTTTGCCCAAGTCATCTG-3'); SOD2, (5'-TGGTTTCAATAAGGAACGGG-3'/5'-GAATAAGGCCTGTTGTTCCT-3'); VEGF, (5'-CCTTGCTGCTCTACCTCCAC-3'/5'-ATGATTCTGCCCTCCTCCTT-3'; β-Actin, (5'-GAAGATCAAGATCATTGCTCCTC-3'/5'-ATCCACATCTGCTGGAAGG-3'). The expression levels were normalized to the housekeeping β-Actin and fold change was calculated using the 2^{-ΔΔCt} method.

For assessing of the MCP-1 levels secreted by the organoids the human MCP-1 Standard TMB ELISA development kit (Peprotech) according to the manufacturer's protocol was used. A preferred method for validating the Enzyme-Linked Immunosorbent Assay (ELISA), includes the following steps:
i) plating the cell supernatants in duplicates and incubating with MCP-1 detection antibody and HRP-streptavidin conjugate.
ii) Performing the stopping colour development reaction,
iii) measuring the absorbance at 450 nm using a Synergy HT microplate reader (Agilent) and normalized by the protein content of each sample ((ng/ml)/µg of protein).

A further exemplification embodiment is described in detail in the following paragraphs.

In an exemplificative embodiment, the retinal organoids for the human 3D-model are generated from hiPSC line IMR90-4 where organoid differentiation was performed until stage 2 of differentiation (80 days-120 days) and immunostaining analysis at day d120 markedly revealed a dense packed apical layer formed by photoreceptors precursors (CRX-positive) (Fig. 1A, images b, f), an internal cell layer of RGCs positive for gamma-synuclein (gSyn) and a defined outer limiting membrane positive for Phalloidin (Fig. 1A, images c, g). Furthermore, there was evidence of interneurons, specifically amacrine and developing horizontal cells detected by AP2α and differentiating Müller glia progenitors' cells shown by vimentin immunolabeling (Fig. 1A, images d, h). At this differentiation stage (d80-d120), the cellular organization and the laminated neuroretina observed indicate that organoids recapitulate the progression of the human retinogenesis. Moreover, gene expression analysis revealed an increasing expression of the RGCs marker (BRN3B) after 40 and 80 days of differentiation and the photoreceptor precursors markers (CRX and RCVRN) from 40 days of differentiation until 120 days (Fig. 1B-D). As expected, this data confirms that the onset of BRN3B expression occurs earlier than photoreceptors differentiation. Thus, retinal organoids after day 100 present mostly retinal cells that are affected in DR and other retinal degenerative diseases, such as glaucoma, providing an interesting physiologically-relevant model for disease modelling and drug screening.

An exemplificative embodiment of the present invention with the purpose of mimicking diabetic conditions, retinal organoids with 100 days of differentiation were subjected to either normal (19 mM) or high glucose concentrations (50 mM and 75 mM) for 1 to seven days, preferably 6 days (Fig. 2A). The nuclei staining with DAPI of cross-sections revealed a significant increase in the percentage of pyknotic nuclei (characteristic of apoptotic cells), on the organoids exposed to 75 mM of glucose in comparison with control and 50 mM of glucose (Fig. 2B, images a-d and 2C). Furthermore, 75 mM of Mannitol which was used as an osmolarity control, did not show significant differences in the number of pyknotic nuclei in comparison with the control condition. In parallel, neurodegeneration induced by high glucose conditions was evaluated by assessing the percentage of positive cells for Fluoro-Jade-C (FJ-C) staining, a dye that stains degenerating mature neurons, including apoptotic, necrotic, and autophagic cells, but also is a reliable marker of degenerating immature neurons and neural stem/precursor cells. It was observed a significantly increased percentage of FJ-C positive cells in cross-sections of organoids exposed to 75 mM compared to the control condition (Fig. 2B, images e-h and 2D). Overall, this data shows that the exposition exposure of the retinal organoids of the present invention to 75 mM of glucose, for 6 days induces cell death and neurodegeneration, as reported in the early stages of DR in both animal models and in eyes of diabetic patients, thereby corroborating the parallelism between the artificial model of the present invention and the eye of a patient with a particular disorder.

An exemplificative embodiment where high glucose exposure affected the different cell type of the retinal organoids is hereby described. AP2α belongs to a family of AP2 transcription factors and is normally used as a marker of amacrine cells but it is known to be expressed in developing horizontal cells as well, as is the case of retinal organoids at day 100. Indeed, the number of AP2α positive cells per section decreased about 50% in retinal organoids exposed to high glucose conditions, indicating a reduction of amacrine and horizontal cells, (Fig. 3A, images a-c and 3B). Moreover, the number of BRN3a positive cells (RGCs) per section presented a significant 2-fold decrease at higher glucose conditions (75mM) (Fig. 3A, images d-f and 3C), while the number of OTX2 positive cells (photoreceptor progenitor cells) has not changed (Fig. 3A, images g-l and 3D). These data reinforce that the treatment of retinal organoids for 6 days with high glucose concentrations is enough to reproduce loss of specific populations in DRN.

Similarly, an exemplificative embodiment where high glucose conditions induce inflammation in retinal organoids is described. Müller cells, the main glial cells in the retina, are thought to be a major source of inflammatory factors in DR. Müller cells express and secrete several growth factors and cytokines that alter the function and survival of retinal neurons and capillary cells. These cells could be responsible for production and secretion of several inflammatory mediators including vascular endothelial growth factor (VEGF), monocyte chemoattractant protein-1 (MCP-1), tumour necrosis factor-α (TNF-α), interleukin-1β (IL-1β), interleukin-6 (IL-6) [48]. Functional Müller cells contribute to the removal of the extracellular glutamate and production of glutamine essential for retinal neurons. Under pathological conditions, such as glaucoma or ischemia, the dysregulation of Müller cells showed a decrease in the glutamate uptake and the glutamine release caused by the impairment of glutamine synthetase. In contrast, in DR and optic nerve crush, no changes or a slight increase of glutamine synthetase were detected. Results showed that, at d100, retinal organoids present Müller glia progenitors as indicated by immunostaining of cross-sections with the corresponding markers vimentin and Nestin (Fig. 4A). Interestingly, both immunostaining signals tend to increase under high glucose conditions and cytoskeleton morphology changes can also be observed. Additionally, as an indicator of glial function, the protein levels of glutamine synthetase was 4-fold increase in organoids exposed to 50 mM compared to the control conditions, while the levels of glutamine, quantified by the glutamine release assay, was significantly increased in organoids exposed to 75 mM of glucose compared to the control conditions (from approximately 0.15 to 0.25 mmol/L/ug) (Fig. 4B and C). In this model, the increase of glutamine synthetase could be due to an increase of the defence against oxidative stress and as a primary response to protect against neuronal degeneration in high glucose conditions. Moreover, given the important role of HIF-1α as a regulator of cellular oxygen homeostasis that facilitates the adaptation to hypoxia in the DR pathogenesis, and responsible for the expression of pro-inflammatory cytokines and VEGF, it was decided to measure HIF-1α and VEGF expression. Interestingly, the present results showed that HIF-1α expression does not change, although VEGF expression was almost 2-fold increase in retinal organoids under high glucose conditions (Fig. 4D). In addition, pro-inflammatory factors such as IL-1β and MCP-1 were significantly altered. Indeed, increased gene expression of IL-1β was significantly observed at 75 mM condition (Fig. 4E) and MCP-1 secretion, that is known to be produced by glial cells in diabetic patients, showed higher levels at 50 mM conditions (Fig. 4F). Altogether, the present results show that under the experimental design, retinal organoids at d100 already present markers of gliosis and inflammation, which are important features of DRN.

Similarly, an exemplificative embodiment where high glucose conditions induce oxidative stress in retinal organoids is described. Oxidative stress is a hallmark of DR where the reactive oxygen species (ROS) production is known to be increased due to chronic high glucose effect on the activation of the secondary pathways like the polyol and hexosamine pathways, besides the overproduction of AGEs which in turns also increases ROS, generating a vicious cycle. To assess if there was oxidative stress, the production of ROS levels assessed by the 2'-7'-dichlorofluorescin diacetate (DCF-DA) probe is evaluated. Through DCF-DA fluorescence intensity measured by confocal imaging, a significant production of intracellular ROS was observed in organoids treated at high glucose conditions (75 mM) compared to control and 50 mM conditions (Fig. 5A). In addition, the gene expression and protein levels of key enzymes involved in the first line defence to oxidative stress, such as superoxide dismutases (SODs), Catalase and GPX1 were analysed. Interestingly, a significant increase of the protein levels of SOD1 and SOD2 was observed in retinal organoids exposed to high glucose conditions, indicating that a post-transcription regulation of these enzymes is occurring (Fig.5D, E). Furthermore, alterations on Glutathione Peroxidase 1 (GPX1) expression were not detected in high glucose conditions (Fig. 5B), while Catalase protein levels were significantly increased at 50 mM conditions (Fig.SF). Overall, this data indicates that the antioxidant response is being induced with the purpose of controlling ROS action, in retinal organoids treated with high glucose concentrations.

Similarly, an exemplificative embodiment where high glucose conditions induce oxidative stress in retinal organoids is described. The phosphatidylinositol3-kinase (PI3K) / Akt / mammalian target rapamycin (mTOR) signalling pathway is a well-known central payer on large number of biological events related to cell growth, division and metabolism. Moreover, dysregulation in mTOR is associated with various diseases such as obesity, diabetes, cancer and neurological diseases. There is significant evidence in DR that the PI3K/Akt/mTOR signalling pathway is connected to various mechanisms associated with disease progression, including oxidative stress, inflammation, hypoxia, angiogenesis, and proliferation. Therefore, the expression levels of mTOR and AKT were evaluated and observed a significant increase in the expression of both genes in retinal organoids exposed to 75 mM of glucose, compared to the control (19mM) and 50 mM conditions (Fig.6A). The AKT protein and its S473 phosphorylation levels were not significantly increased in the tested conditions (Fig. 6B, C). However, the phosphorylation levels of the major downstream effector of mTOR, the ribosomal S6 kinase (S6) at Ser235/236, were significantly increased in retinal organoids exposed to 75 mM of glucose, compared to the control (19mM) and 50 mM conditions (Fig. 6B, D).

Advantages of the aforementioned embodiments include that retinal organoids represent a more advantageous model to study this early phase of DR, dissecting DRN, without the interference of the vascular system.

Furthermore, by exposing the retinal organoids to high glucose conditions, the several phenotypes known to occur in the early phase of DR, were recreated. These include the induction of glial reactivity and inflammation, retinal stress and the alteration of the morphology and immunoreactivity of Vimentin and Nestin.

The more general and advantageous configurations of the present invention are described above. Such configurations are detailed below in accordance with other advantageous and/or preferred embodiments of implementation of the present invention.

As will be clear to one skilled in the art, the present invention should not be limited to the embodiments described herein, and a number of changes are possible which remain within the scope of the present invention.

Of course, the preferred embodiments shown above are combinable, in the different possible forms, being herein avoided the repetition all such combinations.

## Claims

1. *In vitro* human 3D cellular model comprising induced pluripotent stem cells (iPSCs) expressing BRN3B, CRX, RCVRN, VEGF and IL-1β for treating or preventing a disease or disorder of the eye in a patient, wherein the 3D cellular model mimics an early stage of said disease or disorder of the eye.

2. 3D cellular model according to the previous claim for treating or preventing Diabetic Retinopathy.

3. 3D cellular model according to the previous claim for treating glaucoma.

4. 3D cellular model according to claims 1-3 comprising from 5-10 AP2α+ cells/section (%) of amacrine cells when exposed to levels of glucose from 50-70 mM and MCP-1 secretion from 1.2-2 ng/ml MCP-1/µg protein and fluorescence intensity levels of intracellular reactive oxygen species from 11-14 arbitrary units.

5. Process for obtaining a pharmaceutical preparation comprising the step of providing a 3D cellular model according to any of the previous claims.

6. Process for obtaining the 3D cellular model of claims 1-4 comprising the step of obtaining differentiated retinal organoids from human iPSCs to generate an early-stage model of a given eye disease or disorder, preferably diabetic retinopathy.

7. Process according to the previous claim comprising the step of lifting the hiPSCs, preferably at around 80% of confluence, and splitting the cells in a culture medium, preferably a medium supplemented with an inhibitor of the ROCK pathway.

8. Process according to claims 6-7 comprising the step of growing the cells as aggregates or embryoid bodies.

9. Process according to previous claims 6-8 comprising the step of conditioning the organoids in 3D- retinal differentiation medium.

10. 3D model of claim 1-4 for drug screening or gene therapy and pre-clinical and clinical research.

11. 3D model of claim 1-4 for preventing and treating neurodegeneration and inflammation, preferably in early DR.

12. 3D model of claim 1-4 for personalized medicine treatment.

13. System comprising the 3D model of claims 1-4, preferably comprising a microfluidic device, microglia, a vascular system and/or retinal pigments epithelium on retinal organoids.

14. System according to the previous claim being an organoid-on-a-chip.

15. Kit comprising the 3D model of claims 1-4 for diagnosis of a disorder of the eye, preferably diabetic retinopathy or glaucoma.
